(19)

Europäisches
Patentamt
European
Patent Office
Office européen
des brevets

(11) **EP 4 227 686 A1**

(12) **EUROPEAN PATENT APPLICATION**
published in accordance with Art. 153(4) EPC

(43) Date of publication:
**16.08.2023 Bulletin 2023/33**

(21) Application number: **21877738.1**

(22) Date of filing: **08.10.2021**

(51) International Patent Classification (IPC):
*G01N 33/84* (2006.01)       *C07K 14/705* (2006.01)
*C07K 16/28* (2006.01)       *C07K 16/46* (2006.01)
*G01N 33/15* (2006.01)

(52) Cooperative Patent Classification (CPC):
**C07K 14/705; C07K 16/28; C07K 16/46;**
**G01N 33/15; G01N 33/84**

(86) International application number:
**PCT/JP2021/037296**

(87) International publication number:
**WO 2022/075439 (14.04.2022 Gazette 2022/15)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **08.10.2020   JP 2020170478**

(71) Applicants:
• **National University Corporation Tokai National**
  **Higher Education and Research System**
  **Nagoya-shi, Aichi 464-8601 (JP)**
• **Perseus Proteomics Inc.**
  **Tokyo 153-0041 (JP)**

(72) Inventors:
• **KIYOI, Hitoshi**
  **Nagoya-shi, Aichi 464-8601 (JP)**
• **ISHIKAWA, Yuichi**
  **Nagoya-shi, Aichi 464-8601 (JP)**
• **NAKASHIMA, Marie**
  **Nagoya-shi, Aichi 464-8601 (JP)**

• **OHIRA, Yuta**
  **Tokyo 153-0041 (JP)**
• **OYA, Junpei**
  **Tokyo 153-0041 (JP)**
• **NOMURA, Fumiko**
  **Tokyo 153-0041 (JP)**
• **KATSUMI, Keiko**
  **Tokyo 153-0041 (JP)**
• **SAKAMOTO, Aya**
  **Tokyo 153-0041 (JP)**
• **UKAI, Yoshinori**
  **Tokyo 153-0041 (JP)**
• **MATSUURA, Tadashi**
  **Tokyo 153-0041 (JP)**

(74) Representative: **Hoffmann Eitle**
  **Patent- und Rechtsanwälte PartmbB**
  **Arabellastraße 30**
  **81925 München (DE)**

Remarks:
The complete document including Reference
Table(s) and the Sequence Listing(s) can be
downloaded from the EPO website

(54) **METHOD FOR DETERMINING SENSITIVITY OR MEDICINAL EFFECT OF ANTI-TRANSFERRIN RECEPTOR ANTIBODY**

(57)    It is an object of the present invention to provide a method for determining the medicinal effect or sensitivity of an anti-transferrin receptor antibody. According to the present invention, provided is a method for determining the medicinal effect or sensitivity of an anti-human transferrin receptor antibody having an action to inhibit the binding between human transferrin and a human transferrin receptor, wherein the method uses an intracellular iron content as an indicator.

EP 4 227 686 A1

[Fig. 4]

Relationship between intracellular iron content and GI50 value

**Description**

Technical Field

The present invention relates to a method for determining the medicinal effect or sensitivity of an anti-transferrin receptor antibody.

Background Art

Transferrin receptor (TfR) is a type II membrane protein that is expressed on the surface of a cell. TfR binds to transferrin (TF) serving as a ligand, so that it imports iron into a cell and maintains the survival and division of the cell. It has been reported that the expression level of TfR is low in a majority of normal cells, and that TfR is expressed at a low level in several types of cells such as, for example, skin epidermal basal cells and small intestinal epithelial cells (Non-Patent Documents 1 to 3). However, since there is a high demand for iron uptake in erythroblast cells and placental trophoblast cells, TfR is highly expressed in these cells (Non-Patent Documents 4 and 5).
Patent Document 1 discloses that the anti-transferrin receptor antibody is useful for the treatment of cancers such as blood cancer. On the other hand, Patent Document 2 discloses an ion uptake inhibitor of inhibiting iron uptake in cells, which comprises the anti-transferrin receptor antibody.

Prior Art Document

Patent Document

  Patent Document 1: International Publication WO2014/073641
  Patent Document 2: International Publication WO2020/105621

Non-Patent Document

  Non-Patent Document 1: P. Ponka, C. N. Lok, The transferrin receptor: role in health and disease, Int. J. Biochem. Cell Biol. 31 (1999) 1111-1137.
  Non-Patent Document 2: D. R. Richardson, P. Ponka, The molecular mechanisms of the metabolism and transport of iron in normal and neoplastic cells, Biochim. Biophys. Acta 1331 (1997) 1-40.
  Non-Patent Document 3: Daniels TR. Delgado T , Rodriguez JA, Helguera G , Penichet ML. The transferrin receptor part I: Biology and targeting with cytotoxic antibodies for the treatment of cancer. Clinical Immunology (2006) 121, 144-158
  Non-Patent Document 4: J. E. Levy, O. Jin, Y. Fujiwara, F. Kuo, N. C. Andrews, Transferrin receptor is necessary for development of erythrocytes and the nervous system, Nat. Genet. 21 (1999) 396-399.
  Non-Patent Document 5: Khatun R, Wu Y, Kanenishi K, Ueno M, Tanaka S, Hata T, Sakamoto H., Immunohistochemical study of transferrin receptor expression in the placenta of pre-eclamptic pregnancy., Placenta. 2003; 24(8-9): 870-6.

Summary of Invention

Object to be Solved by the Invention

As described above, the anti-transferrin receptor antibody has therapeutic effects on diseases such as cancer. However, determination of the medicinal effect or sensitivity of the anti-transferrin receptor antibody has not been known. It is an object of the present invention to provide a method for determining the medicinal effect or sensitivity of an anti-transferrin receptor antibody.

Means for Solving the Object

The present inventors have conducted intensive studies directed towards achieving the aforementioned object. As a result, the present inventors have found that an intracellular iron content can be utilized as an indicator (biomarker) for determining the medicinal effect or sensitivity of an anti-transferrin receptor antibody, thereby completing the present invention.

Specifically, according to the present invention, the following inventions are provided.

<1> A method for determining the medicinal effect or sensitivity of an anti-human transferrin receptor antibody having an action to inhibit the binding between human transferrin and a human transferrin receptor, wherein the method uses an intracellular iron content as an indicator.

<2> The method according to <1>, which is a method for determining the medicinal effect or sensitivity by utilizing the correlation of the intracellular iron content with the medicinal effect of the antibody.

<3> The method according to <1> or <2>, wherein the medicinal effect or sensitivity of the antibody is determined based on the relative value to the iron content in a reference sample.

<4> The method according to any one of <1> to <3>, wherein when the intracellular iron content is lower than the reference value, the medicinal effect or sensitivity of the antibody is determined to be high.

<5> The method according to any one of <1> to <4>, wherein the medicinal effect or sensitivity of the antibody to cancer is determined.

<6> The method according to <5>, wherein the cancer is blood cancer.

<7> The method according to <5> or <6>, wherein the cancer is acute myelogenous leukemia (AML).

<8> The method according to any one of <1> to <7>, wherein the antibody is an antibody recognizing the amino acids at positions 629 to 633 of a human transferrin receptor.

<9> The method according to any one of <1> to <8>, wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

<10> The method according to any one of <1> to <9>, wherein the antibody is an antibody having a heavy chain as set forth in SEQ ID NO: 7 and a light chain as set forth in SEQ ID NO: 8.

Advantageous Effects of Invention

According to the present invention, it becomes possible to determine the medicinal effect or sensitivity of an anti-transferrin receptor antibody.

Brief Description of Drawings

[Fig. 1] Fig. 1 shows the sites of individual TfR mutant fragments, at which point mutations are performed.
[Fig. 2] Fig. 2 shows the reactivity of TfR436 with soluble wild-type TfR (sTfR) and TfR mutant fragments.
[Fig. 3] Fig. 3 shows the Tf-TfR binding inhibitory activity of TfR436.
[Fig. 4] Fig. 4 shows the relationship between an intracellular iron content and a GI50 value in an AML clinical specimen.
[Fig. 5] Fig. 5 shows the antitumor effects of a TfR436 antibody in Kasumi-1 subcutaneous transplantation models.
[Fig. 6] Fig. 6 shows the antitumor effects of a TfR436 antibody in HL60 subcutaneous transplantation models.
[Fig. 7] Fig. 7 shows the life-extending effect of a TfR436 antibody in KO52 caudal vein transplantation models.
[Fig. 8] Fig. 8 shows the life-extending effect of a TfR436 antibody in CCRF-CEM intravenous transplantation models.

Embodiment of Carrying out the Invention

Hereinafter, the present invention will be described in more detail.

Definitions and General Techniques

Unless otherwise specified in the present description, scientific terms used regarding the present invention include meanings that are generally understood by a person skilled in the art. In general, nomenclatures and techniques applied to the cell and tissue culture, molecular biology, immunology, microbiology, genetics, protein and nucleic acid chemistry, and hybridization, which are described in the present description, are well known in the present technical field, and thus, are commonly used.

The methods and techniques of the present invention are carried out in accordance with conventional methods that are well known in the present technical field, in such ways as described in a variety of general reference documents cited and discussed throughout the present description and more specific reference documents, unless otherwise specified.

< Method of the Present Invention >

The method for determining the medicinal effect or sensitivity of an anti-human transferrin receptor antibody having an action to inhibit the binding between human transferrin and a human transferrin receptor according to the present invention is a method of using an intracellular iron content as an indicator. The anti-human transferrin receptor antibody is preferably an antibody that recognizes the amino acids at positions 629 to 633 of a human transferrin receptor. Specifically, when the intracellular iron content is lower than a reference value, the medicinal effect or sensitivity of the antibody can be determined to be high.

The method of the present invention is preferably a method for determining the medicinal effect or sensitivity of the anti-human transferrin receptor antibody, wherein the method utilizes the correlation of the intracellular iron content with the medicinal effect of the antibody. More preferably, the medicinal effect or sensitivity of the antibody can be determined based on the relative value to the iron content in a reference sample. Examples of the reference sample that can be used herein may include reference cells such as K562, and a control sample containing an iron in a reference amount. For example, in a case where K562 cells are used as such a reference sample, when the intracellular iron content in measurement target cells that is as a relative value (comparison value) to the intracellular iron content in the K562 cells is 1 or less, when it is preferably 0.8 or less, is more preferably 0.7 or less, or is further preferably 0.6 or less, the medicinal effect or sensitivity of the antibody can be determined to be high.

Trivalent irons in serum are transported in a state in which the irons bind to transferrin. When such trivalent iron-bound transferrin binds to a transferrin receptor present on the surface of a cell, it is incorporated into the endosome in the cell. The trivalent irons incorporated into the cell together with the transferrin are reduced to divalent irons by metalloreductase STEAP3 in the endosome. The divalent irons are carried to the cytoplasm by DMT1 (divalent metal transporter 1), and are than pooled therein (which is also referred to as "Labile iron pool"). The divalent irons stored in the Labile iron pool are utilized for DNA synthesis or DNA repair, or are utilized for energy generation, heme synthesis and Fe-S cluster biosynthesis in the mitochondria, or are stored as storage irons. Moreover, some divalent irons in the cell are transported from the inner side to the outer side of the cell via ferroportin present in the cell membrane. As described above, intracellular irons are associated with utilization of irons in cells. In the present invention, it has been specifically found that, by using an intracellular iron content as an indicator, the medicinal effect or sensitivity of an antibody can be determined to be high, when the intracellular iron content is lower than a reference value.

In order to determine the medicinal effect or sensitivity of an anti-transferrin receptor antibody according to the method of the present invention, the intracellular iron content in a biological sample (specimen) derived from a subject is measured, and the measurement results are then compared with those of a control level (reference value), so that the medicinal effect or sensitivity of the antibody can be determined.

The subject may be a subject who is in need of a treatment with an anti-transferrin receptor antibody, and as an example, the subject may be a subject having cancer.

The biological sample may be, for example, a biopsy specimen (when the subject is a cancer patient, the biological sample is a specimen containing cancer cells, etc.).

The intracellular iron content can be measured according to an ordinary method, and the measurement can be carried out using a commercially available measurement kit. For example, according to metalloassay iron measurement LS (ferrozine method) (Metallogenics, #FE31M), irons ($Fe^{2+}$ and $Fe^{3+}$) contained in a sample can be quickly quantified using a microplate reader (96-well).

In the present invention, in order to determine the medicinal effect or sensitivity of an anti-transferrin receptor antibody, the intracellular iron content in a biological sample derived from a subject may be measured before administration of the anti-transferrin receptor antibody.

When the intracellular iron content is determined to be lower than a predetermined reference value, it can be determined that the medicinal effect of the anti-transferrin receptor antibody on the subject is high, namely that the subject has sensitivity to the anti-transferrin receptor antibody. Accordingly, the intracellular iron content can be used as a marker for an active and continuous treatment for a patient who is expected to obtain therapeutic effects. On the other hand, when the intracellular iron content is determined to be higher than a predetermined reference value, it can be determined that the medicinal effect of the anti-transferrin receptor antibody on the subject is low, namely that the subject has no sensitivity to the anti-transferrin receptor antibody. The determination may not only be determination based on a specific numerical value as a reference, but it may also be determination based on references other than numerical values that can be grasped through visual sense such as shade of color.

In the present invention, it is preferable to determine the medicinal effect or sensitivity of the antibody to cancer.

The cancer may be any one of solid cancers (for example, lung cancer, colon cancer, stomach cancer, bladder cancer, pancreatic cancer, prostate cancer, liver cancer, cervical cancer, uterine cancer, ovarian cancer, breast cancer, head and neck cancer, skin cancer, etc.), and blood cancers (for example, leukemia, lymphoma, myeloma, etc.). The cancer is preferably blood cancer. The cancer is particularly preferably acute myelogenous leukemia (AML).

< Concerning Antibody >

TfR

Human transferrin receptor (TfR) is a single-pass transmembrane protein (SEQ ID NO: 9) composed of 760 amino acids, and it is encoded by human chromosome 3. This protein has also been known as a CD71 antigen, and it is considered that this protein is associated with incorporation of iron into cells and cell growth. The structure of the TfR of the present invention is not particularly limited, and thus, the TfR of the present invention always means human TfR including all of a monomer, a polymer, an intact form expressed on a cell membrane, a soluble form constituted in an extracellular region, a truncated form, a mutation form caused by genetic mutation, deletion, etc., and a form that has undergone posttranslational modification by phosphorylation or the like.

React and Reactivity

The terms "react" and "reactivity" have the same meanings in the present description, unless otherwise specified. That is, these terms mean that an antibody recognizes an antigen. The antigen used herein may be any of an intact TfR expressed on a cell membrane, a truncated form, and a soluble form. In addition, the antigen may be either a TfR having a three-dimensional structure or a modified TfR. Examples of a means for examining reactivity include flow cytometry (FACS), enzyme-linked immunosorbent assay (ELISA), Western blotting, microfluorescence measuring technique (FMAT), surface plasmon resonance (Biacore), immunostaining, and immunoprecipitation.

The antibody used in flow cytometry may be either an antibody labeled with a fluorescent substance such as FITC or with biotin, or an unlabeled antibody. A fluorescently-labeled avidin, a fluorescently-labeled anti-human immunoglobulin antibody, or the like is used, depending on the presence or absence of labeling of the antibody used and the type thereof. Reactivity can be evaluated by adding a sufficient amount of anti-TfR antibody (generally having a final concentration of 0.01 to 10 μg/mL) to an analyte, and then by comparing the obtained reactivity with the reactivity with a negative control antibody or a positive control antibody.

Antibody

In the present description, the following abbreviations (in the parentheses) are used in accordance with the customs, as necessary.

Heavy chain (H chain), light chain (L chain), heavy chain variable region (VH), light chain variable region (VL), complementarity determining region (CDR), first complementarity determining region (CDR1), second complementarity determining region (CDR2), third complementarity determining region (CDR3), heavy chain first complementarity determining region (VH CDR1), heavy chain second complementarity determining region (VH CDR2), heavy chain third complementarity determining region (VH CDR3), light chain first complementarity determining region (VL CDR1), light chain second complementarity determining region (VL CDR2), and light chain third complementarity determining region (VL CDR3).

In the present description, the term "antibody" has the same definitions as immunoglobulin, and should be understood as generally known in the present technical field. Specifically, the term "antibody" is not limited by any given specific method for producing the antibody. For example, the term "antibody" includes, but is not limited to, a recombinant antibody, a monoclonal antibody, and a polyclonal antibody.

In the present description, the term "human antibody" is used to mean any given antibody, in which the sequences of a variable region and a constant region are human sequences. This term includes antibodies which have human sequences and are modified, for example, to remove cysteine that may cause a possible decrease in immunogenicity, an increase in affinity, and undesirable folding. This term also includes antibodies produced in non-human cells by recombination, which enable glycosylation that is not specific to human cells. These antibodies can be prepared in various ways.

In the present description, the term "humanized antibody" means a non-human-derived antibody, in which amino acid residues characteristic for a non-human antibody sequence are substituted with residues found in positions corresponding to those of a human antibody. This "humanization" process is considered to reduce the immunogenicity of the obtained antibody in human. It would be understood that a non-human-derived antibody can be humanized using a technique well known in the present technical field. Please refer to, for example, Winter et al., Immunol. Today 14:

43-46 (1993). The target antibody can be produced by an engineering approach via a recombination DNA technique of substituting CH1, CH2, CH3, a hinge domain, and/or a framework domain with those of the corresponding human sequence. For example, WO92/02190, and U. S. Patent Nos. 5,530,101, 5,585,089, 5,693,761, 5,693,792, 5,714,350 and 5,777,085 can be referred. In the present description, the term "humanized antibody" includes a chimeric human antibody and a CDR-grafted antibody, within the definitions thereof.

The sequence of a framework region (FR) in a variable region of the antibody is not particularly limited, unless it substantially affects the specific binding ability of the antibody to the corresponding antigen. The FR region of a human antibody is preferably used, but it is also possible to use FR regions of animal species other than humans (e.g. a mouse or a rat).

In one aspect, the antibody comprises a constant region as well as a variable region (e.g. IgG antibody). The sequence of such a constant region is not particularly limited. For example, the constant region of a known human antibody can be used. The heavy chain constant region (CH) of a human antibody is not particularly limited, as long as it belongs to a human immunoglobulin (hereinafter referred to as "hIg"). Those of hIgG class are preferable, and any one of subclasses belonging to hIgG class, such as hIgG1, hIgG2, hIgG3 or hIgG4, may be used. On the other hand, the light chain constant region (CL) of a human antibody is not particularly limited, as long as it belongs to hIg, and those of $\kappa$ class or $\lambda$ class can be used. In addition, constant regions of animal species other than humans (e.g. a mouse or a rat) can also be used.

In the present description, the term "modified body" or "modified antibody" is used to mean that the amino acid sequence of the variable region (CDR sequences and/or FR sequences) of a parent antibody comprises a substitution, deletion, addition and/or insertion of one or multiple amino acids.

In the present description, the "parent antibody" means a TfR436 antibody that has a VH comprising the amino acid sequence as set forth in SEQ ID NO: 7 and a VL comprising the amino acid sequence as set forth in SEQ ID NO: 8. In the amino acid sequence, one or several (for example, 1 to 8, preferably 1 to 5, more preferably 1 to 3, and particularly preferably 1 or 2) amino acids are deleted, added, substituted and/or inserted. As a method of preparing the amino acid sequence of an antibody having a binding ability to TfR, which has been well known to a person skilled in the art, a method of introducing a mutation into a protein has been known. For instance, such a skilled person could prepare a modified antibody functionally equivalent to an antibody having a TfR-binding activity by appropriately introducing a mutation into the amino acid sequence of the antibody having a TfR-binding activity according to a site-directed mutagenesis (Hashimoto-Gotoh, T, Mizuno, T, Ogasahara, Y, an DNA Kagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492). Thus, an antibody comprising a mutation of one or several amino acids in the variable region or constant region of a parent antibody and having a binding activity to TfR can also be used.

In the present description, the phrase "an activity equivalent to the activity of the parent antibody" is used to mean that the binding activity of a certain antibody to human TfR is equivalent to that of the parent antibody thereof. The term "equivalent" does not necessarily mean the same level of activity. The activity may be increased, or the activity may also be decreased, as long as the antibody has the activity. An antibody having a decreased activity may be an antibody having an activity that is, for example, 30% or more, preferably 50% or more, more preferably 80% or more, further preferably 90% or more, and particularly preferably 95% or more of the activity of the original antibody.

The term "binding activity" means the activity of an antibody to recognize an antigen. This antigen may be an intact TfR expressed on a cell membrane, a truncated form, or a soluble form. In addition, the antigen may be either a TfR having a three-dimensional structure or a modified TfR. Examples of a means for examining the binding activity include flow cytometry (FACS), enzyme-linked immunosorbent assay (ELISA), Western blotting, microfluorescence measuring technique (FMAT), and surface plasmon resonance (Biacore).

The Tf-TfR binding inhibitory activity of an antibody can be measured as follows. That is, a TfR solution is dispensed into a substrate (a 96-well plate, etc.) and is then left at rest to obtain a solid phase, and thereafter, blocking is carried out. Subsequently, an HRP-labeled Tf solution is dispensed into the substrate, and an antibody is further added thereto, so that they are allowed to react with each other at room temperature. Thereafter, the substrate is washed, and a coloring reagent (TMB, etc.) is then added thereto, so that they are allowed to react with each other. After that, the absorbance is measured using a plate reader. By performing the above-described operations, the Tf-TfR binding inhibitory activity of the antibody can be evaluated.

The antibody is not limited by its origin, and it may be an antibody derived from any animal, such as a human antibody, a mouse antibody, or a rat antibody. Also, the present antibody may be a chimeric antibody or a humanized antibody. One preferred aspect of the antibody of the present invention is a human antibody.

The antibodies may be different from one another in terms of amino acid sequence, molecular weight, isoelectric point, the presence or absence of a sugar chain or the form thereof, etc., depending on the after-mentioned cells or hosts that produce the antibodies, or a purification method. For example, an antibody that undergoes a posttranslational modification on the amino acid sequence described in the present description is included in the present invention. Moreover, an antibody that undergoes a posttranslational modification on a site other than those for the known posttranslational modification is also included in the present invention. Furthermore, when the antibody is allowed to express in prokaryotic cells such as *Escherichia coli,* a methionine residue is added to the N-terminus of the amino acid sequence of the original antibody. Such an antibody may also be used in the present invention. An antibody that undergoes a posttranslational modification on a site other than those for the known posttranslational modification is also included in the present invention.

Production of Antibody

(1) Obtaining of scFv reacting with antigen using phage display library

The antibody can be prepared by several methods known in the present technical field. For example, using a phage display technique, a library comprising a repertoire of antibodies having various affinity for TfR can be provided. Subsequently, such a library can be screened to identify and isolate antibodies against TfR. Preferably, the phage library is a scFv phage display library that is generated using human VL and VH cDNA that has been prepared from mRNA isolated from human B cells. A method of preparing and screening such a library is known in the present technical field. A genetic substance is recovered from phage clones exhibiting reactivity that have been screened using human TfR as an antigen. By analyzing the selected phage gene, the DNA sequences of VH and VL encoding the variable region of a human antibody binding to the antigen can be determined. Using this scFv sequence, IgG is prepared from scFv, so as to obtain a human antibody.

(2) Preparation of IgG from scFv (preparation of human antibody)

An H chain and L chain expression vector is produced, and it is then allowed to express in a host cell. Thereafter, the secreted supernatant is recovered and is then purified, so as to obtain a human antibody. Alternatively, such a human antibody can also be obtained by allowing VH and VL to express in a single vector (tandem type). These methods are well known, and can be carried out with reference to WO92/01047, WO92/20791, WO93/06213, WO93/11236, WO93/19172, WO95/01438, WO95/15388, WO97/10354, etc.

Specifically, DNA encoding VH is ligated to another DNA molecule encoding a heavy chain constant region (CH1, CH2 and CH3), so as to obtain a full-length heavy chain gene. The sequence of a human heavy chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The heavy chain constant region may be the constant region of IgG1, IgG2, IgG3, IgG4, IgA, IgE, IgM or IgD. The most preferred constant region is that of IgG1 or IgG2. The constant region sequence of IgG1 may include any given various alleles or allotypes known to be generated among different individuals, such as Gm (1), Gm (2), Gm (3) or Gm (17). These allotypes correspond to a substitution of amino acids naturally-occurring in the constant region of IgG1.

DNA encoding VL is ligated to another DNA molecule encoding the light chain constant region CL, so as to obtain a full-length L chain gene (and a Fab light chain gene). The sequence of a human light chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The light chain constant region may be the constant region of κ or λ. The κ constant region may include any given various alleles known to be generated among different individuals, such as Inv (1), Inv (2) or Inv (3). The λ constant region may be derived from any one of the three λ genes.

The thus obtained DNA encoding an H chain or L chain is inserted into a vector to produce an expression vector, and the produced expression vector is then allowed to express in a host cell. Thereafter, the secreted supernatant is recovered and purified to obtain a human antibody. Examples of the expression vector include a plasmid, retrovirus, adenovirus, adeno-associated virus (AAV), plant viruses such as cauliflower mosaic virus or tobacco mosaic virus, a cosmid, YAC, and EBV-derived episome. An expression vector and an expression regulatory sequence are selected, so that they are suitable for a host cell used for expression. An antibody light chain gene and an antibody heavy chain gene can be inserted into different vectors, or the two genes can also be inserted into a single expression vector. An antibody gene is inserted into an expression vector by a standard method (for example, ligation of a complementary restriction site on an antibody gene fragment to a vector, or blunt-ended ligation applied when no restriction sites are present).

A favorable vector encodes a functionally complete human CH or CL immunoglobulin sequence having a suitable restriction site, which has been produced by an engineering approach such that any given VH or VL sequence can be easily inserted and then expressed therein, as described above. In such a vector, splicing generally takes place between a splice donor site in the inserted J region and a splice acceptor site preceding a human C domain, or such splicing also takes place in a splice region existing in a human CH exon. Polyadenylation and transcription termination take place in a natural chromosomal site downstream of a coding region. A recombinant expression vector can also encode a signal peptide that promotes the secretion of an antibody chain derived from a host cell. An antibody chain gene can be cloned into a vector, such that a signal peptide can be ligated in-frame to the amino terminus of an immunoglobulin chain. The signal peptide may be either an immunoglobulin signal peptide or a heterogeneous signal peptide (namely, it may be a non-immunoglobulin protein-derived signal peptide).

An expression vector used for the antibody of the present invention may also have sequences such as a sequence for regulating replication of the vector in a host cell (e.g. a replication origin) or a selective marker gene sequence, as well as an antibody gene and a regulatory sequence. The selective marker gene promotes selection of a host cell into which a vector has been introduced. For instance, the selective marker generally imparts resistance to drugs such as G418, hygromycin or methotrexate to a host cell into which the vector has been introduced. Preferred selective marker genes include a dihydrofolate reductase (DHFR) gene (used in selection/amplification of methotrexate as a dhfr-host cell), a neomycin phosphotransferase gene (used in selection of G418), and a glutamate synthase gene.

A host cell is transformed with an antibody gene expression vector produced by the above-described method. Any type of cell may be used as a host cell, as long as it can produce the present antibody. Examples of such a host cell include bacteria, yeast, animal cells, insect cells, and plant cells. Among these cells, animal cells are preferable. Examples of the animal cells include Chinese hamster ovary cells CHO/dhfr(-) and CHO/DG44, monkey-derived cells COS (A. Wright & S. L. Morrison, J. Immunol. 160, 3393-3402 (1998)), and SP2/O cells (mouse myeloma) (K. Motmans et al., Eur. J. Cancer Prev. 5, 512-5199 (1996), R. P. Junghans et al., Cancer Res. 50, 1495-1502 (1990)). For transformation, a lipofectin method (R. W. Malone et al., Proc. Natl. Acad. Sci. USA 86, 6007 (1989), P. L. Felgner et al., Proc. Natl. Acad. Sci. USA 84, 7413 (1987)), an electroporation method, a calcium phosphate method (F. L. Graham & A. J. van der Eb, Virology 52, 456-467 (1973)), a DEAE-Dextran method, and the like are preferably applied.

A transformant is cultured, and a human antibody is then separated from the cells of the transformant or a culture medium thereof. For separation/purification of the antibody, methods such as centrifugation, ammonium sulfate fractionation, salting-out, ultrafiltration, affinity chromatography, ion exchange chromatography and gel filtration chromatography can be used by appropriately combining them.

Antibody Fragments

An antibody fragment can be produced based on the present antibody, or based on the sequence information of a gene encoding the present antibody. Examples of the antibody fragment include Fab, Fab', F(ab')$_2$, scFv, and dsFv antibodies.

Fab is obtained by digesting IgG by papain in the presence of cysteine. It is an antibody fragment with a molecular weight of approximately 50,000, which is constituted with L chain and H chain variable regions, and an H chain fragment consisting of a CH1 domain and a portion of a hinge region. In the present invention, the above-described antibody can be obtained by papain digestion. In addition, Fab can also be prepared by incorporating DNA encoding a portion of the H chain and the L chain of the above-described antibody into a suitable vector, then performing transformation with the resulting vector, and then obtaining it from the transformant .

Fab' is an antibody fragment with a molecular weight of approximately 50,000, which is obtained by cleaving a disulfide bond between the H chains of the below-mentioned F(ab')$_2$. In the present invention, Fab' can be obtained by digesting the above-described antibody by pepsin, and then cleaving a disulfide bond with a reducing agent. In addition, as with Fab, Fab' can also be prepared by genetic engineering using DNA encoding the Fab'.

F(ab')$_2$ is an antibody fragment with a molecular weight of approximately 100,000, which is obtained by binding, via a disulfide bond, one fragment (Fab') constituted with L chain and H chain variable regions and an H chain fragment consisting of a CH1 domain and a portion of a hinge region, to the other fragment (Fab'). In the present invention, F(ab')$_2$ can be obtained by digesting the above-described antibody by pepsin. In addition, as with Fab, F(ab')$_2$ can also be prepared by genetic engineering using DNA encoding the F(ab')$_2$.

scFv is an antibody fragment obtained by ligating the C-terminus of one chain of Fv consisting of an H chain variable region and an L chain variable region to the N-terminus of the other chain thereof, using a suitable peptide linker, so as to form a single chain. (GGGGS)$_3$ having high flexibility can be used, for example, as such a peptide linker. For instance, DNA encoding the H chain variable region and L chain variable region of the above-described antibody and DNA encoding a peptide linker are used to construct DNA encoding a scFv antibody, and the thus constructed DNA is then incorporated into a suitable vector. Thereafter, scFv can be prepared from a transformant obtained by transformation with the aforementioned vector.

dsFv is an Fv fragment obtained by introducing a Cys residue into a suitable site in each of an H chain variable region and an L chain variable region, and then stabilizing the H chain variable region and the L chain variable region by a disulfide bond. The site in each chain, into which the Cys residue is to be introduced, can be determined based on a conformation predicted by molecular modeling. In the present invention, for example, a conformation is predicted from the amino acid sequences of the H chain variable region and L chain variable region of the above-described antibody, and DNA encoding each of the H chain variable region and the L chain variable region, into which a mutation has been introduced based on such prediction, is then constructed. The thus constructed DNA is incorporated into a suitable vector. Thereafter, dsFv can be then prepared from a transformant obtained by transformation with the aforementioned vector.

Further, it is also possible to ligate the scFv antibody to the dcFv antibody or the like using a suitable linker, or to fuse such an antibody fragment with streptavidin, so as to multimerize the antibody fragment.

The present invention will be described in more detail in the following examples. However, these examples are not intended to limit the scope of the present invention.

Examples

In the following examples, the TfR436 antibody described in paragraphs 0090 and 0091 of International Publication WO2014/073641 was used.

The CDR sequences of the TfR436 antibody will be shown below.

VH CDR1: SYGMH (SEQ ID NO: 1)
VH CDR2: VISYDGSNKYYADSVKG (SEQ ID NO: 2)
VH CDR3: DSNFWSGYYSPVDV (SEQ ID NO: 3)
VL CDR1: TRSSGSIASNSVQ (SEQ ID NO: 4)
VL CDR2: YEDTQRPS (SEQ ID NO: 5)
VL CDR3: QSYDSAYHWV (SEQ ID NO: 6)

The VH sequence and VL sequence of the TfR436 antibody will be shown below.

TfR436 VH (SEQ ID NO: 7)

DVQLVQSGGGVVQPGRSLRLSCAASGFPFKSYGMHWVRQAPGKGLEWVAV
ISYDGSNKYYADSVKGRFTISRDNSKNTLYLQMNSLRGEDTAVYYCARDSNF
WSGYYSPVDVWGQGTTVTVSS

TfR436 VL (SEQ ID NO: 8)

NFMLTQPHSVSESPGKTVTISCTRSSGSIASNSVQWYQQRPGSAPITVIYEDTQ
RPSGVPDRFSGSIDSSSNSASLTISGLQTEDEADYYCQSYDSAYHWVFGGGTK
LAVL

Example 1: Identification of binding site of TfR436 antibody

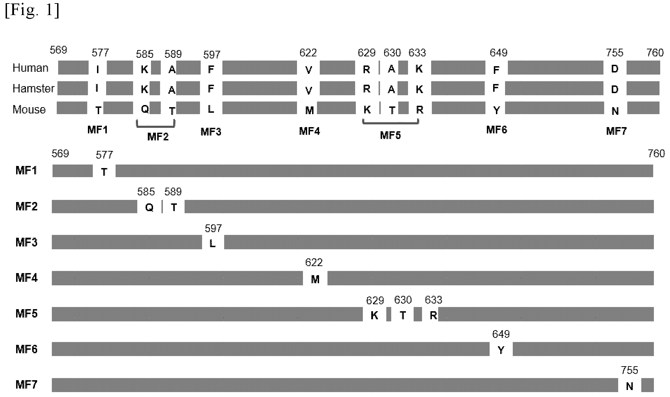

The TfR436 antibody did not cross-react with mouse TfR, but it exhibited cross-reactivity with hamster TfR. The amino acid sequence of a transferrin (TF)-binding site (the amino acids at positions 569 to 760) in TfR was aligned. The amino acids of a human TfR sequence, which were the same as hamster but were different from mouse, were picked up. The picked amino acids were subjected to point mutation, as shown in Fig. 1, so that soluble TfR mutant fragments were produced.

(1) Production of soluble wild-type TfR (sTfR) and TfR mutant fragments (MF1 to MF7)

A human TfR extracellular domain (the amino acids at positions 89 to 760) or each TfR mutant fragment (MF1 to MF7) as shown in Fig. 1, and a nucleotide sequence encoding AAARGGPEQKLISEEDLNSAVDHHHHHH (SEQ ID NO: 10) were totally synthesized. A neomycin resistance gene and a DHFR gene were incorporated into the expression vector pCAGGS (Non-Patent Document 2.: Niwa et al. 1991), and thereafter, the synthesized genes were each inserted into the multicloning site of the obtained expression vector, so as to produce a pCAGGS-Neo-DHFR-sTFR-myc-his expression plasmid. Using Expifectamine (Thermo Fisher Scientific), the Expi293 cells (Thermo Fisher Scientific) were transfected with the above-described plasmid, and were then cultured at 37°C in 8% $CO_2$ at 135 rpm for 5 days. Thereafter, a culture supernatant was recovered by centrifugation. A HisTrapHP (Cytiva) column was connected with AKTA prime (Cytiva), and sTfR or MF1 to MF7 were then purified using 20 mM Imidazole/DPBS as a binding buffer and 500 mM Imidazole/DPBS as an elution buffer. The eluted protein was subjected to buffer exchange with 30 mM HEPES, 5% trehalose, pH 7.2, by using a Zeba spin column (Thermo Scientific).

(2) Identification of binding site of TfR436 antibody

The thus purified sTfR or MF1 to MF7 were diluted with PBST (Phosphate Buffered Saline with Tween 20, TaKaRa), and the diluted solutions were prepared at 7 stages by 3-fold dilution from 600 ng/mL. Thereafter, the diluted solution was dispensed into Ni-NTA HisSorb Strips 96-well plate (QIAGEN) in an amount of 100 μL/well, and the plate was then placed on a shaker, followed by performing a reaction at room temperature. One hour later, the reaction mixture was washed with PBST Buffer 5 times, and the TfR436 antibody (1 μg/mL) was dispensed into the 96-well plate in an amount of 100 μL/well. The plate was placed on a shaker, and a reaction was then performed at room temperature for 1 hour. Thereafter, the reaction mixture was washed with PBS-T Buffer 5 times, and a 50,000-fold diluted secondary antibody, F(ab')2 Fragment Anti-Human IgG Fcγ (Jackson Immuno Research), was then dispensed into the 96-well plate in an amount of 100 μL/well, followed by performing a reaction at room temperature for 1 hour. The reaction mixture was washed with PBST Buffer 5 times, and TMB Soluble Reagent (High Sensitivity) (Scy Tek) was then dispensed into the plate in an amount of 100 μL/well, followed by performing a reaction at room temperature in a dark place for 3 minutes. After that, TMB Stop Buffer (Scy Tek) was added to the plate in an amount of 100 μL/well, and the obtained mixture was then shaken using a shaker for 1 minute. Thereafter, the absorbance at 450 nm (ref. 620 nm) was measured using a plate reader.

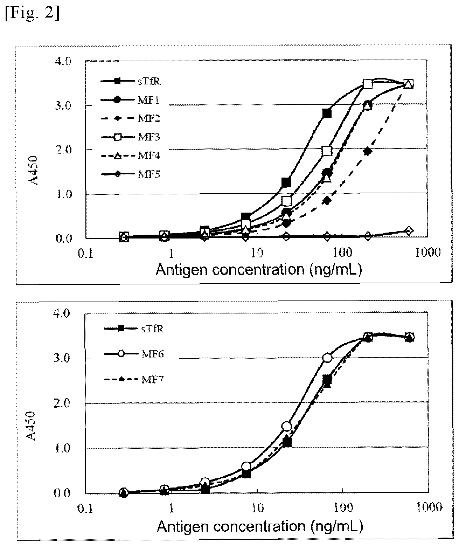

As a result, as shown in Fig. 2, a reduction in the reactivity of the TfR436 antibody with the TfR mutant fragment MF5 was observed, but a reduction in the reactivity of the TfR436 antibody with other mutant fragments was not observed. That is to say, when the amino acids at positions 629, 630 and 633 of TfR are substituted with other amino acids, the TfR436 antibody cannot recognize the TfR. Thus, it was suggested that the amino acids at positions 629 to 633 of TfR should be an epitope recognized by the TfR436 antibody.

Example 2: Comparison between TfR436 antibody and comparative antibodies in terms of Tf-TfR binding inhibition

(1) Production of comparative antibody A24

Patent Document US2008/0193453 discloses an A24 antibody reacting against human TfR. In order to compare the TfR436 antibody with the A24 antibody, the deposited hybridomas were acquired, and the antibody was produced. Specifically, the hybridomas were seeded into RPMI1640 (Thermo Fisher Scientific) supplemented with 10% FBS at a cell concentration of 1 to 2 × $10^5$/mL, and were then cultured in a 5% $CO_2$ incubator at 37°C. After completion of an expansion culture, the cells were recovered by centrifugation, and were then washed with PBS twice. Thereafter, the resulting cells were further subjected to an expansion culture in a serum-free medium Cosmedium 005 (Cosmo Bio Co., Ltd.) and 0.5% Nutridoma-CS (Roche) to result in a volume of 550 mL. Five days after the cells became confluent, a culture supernatant was recovered by centrifugation.

The recovered supernatant was applied onto a protein A carrier (Ab-Capcher ExTra: ProteNova), and an antibody binding to protein A was eluted with a 0.1 M glycine-HCl buffer (pH 2.7), and then, was rapidly neutralized with a 1 M Tris-HCl buffer (pH 8.5). Thereafter, using an Ultracell Ultrafiltration Disk (Merck Millipore), the buffer was exchanged with PBS.

(2) Comparison between TfR436 antibody and antibodies of other companies in terms of Tf-TfR binding inhibition

The sTfR described in Example 1 was adjusted to a concentration of 5.0 μg/mL by addition of PBST, and the diluted solution was then dispensed into MaxiSorp 96-well plate (Nunc) in an amount of 100 μL/well. Thereafter, it was left at rest at 4°C overnight to obtain a solid phase. On the following day, the solid phase solution was discarded, and 100% Block Ace (DS Pharma Biomedical) was added to the plate in an amount of 200 μL/well, followed by leaving the obtained mixture at rest at room temperature so as to carry out blocking. One hour later, the reaction mixture was washed with PBST Buffer 5 times, and HRP-labeled Tf (2 μg/mL) was then dispensed into the plate in an amount of 50 μL/well. Thereafter, the TfR436 antibody, the A24 antibody (2-fold dilution series from 10 μg/mL), or holo-Tf (Sigma) (2-fold dilution series from 300 μg/mL) was further added to the mixture in an amount of 50 μL/well. The obtained mixture was reacted at room temperature for 1 hour, and was then washed with PBST Buffer 5 times. Thereafter, TMB Soluble Reagent (High Sensitivity) was dispensed into the plate in an amount of 100 μL/well, and the obtained mixture was then reacted at room temperature in a dark place. Twenty five minutes later, TMB Stop Buffer was added to the plate in an amount of 100 μL/well, and the obtained mixture was shaken with a shaker for 1 minute. Thereafter, the absorbance at 450 nm (ref. 620 nm) was measured.

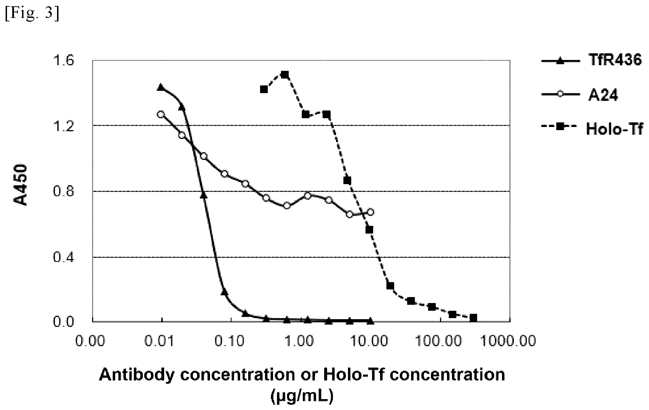

As a result, as shown in Fig. 3, the TfR436 antibody completely inhibited the binding of Tf-TfR at a significantly low dose (100 ng/mL). In contrast, the A24 antibody could not completely inhibit the binding of Tf-TfR, even though it was used at a dose of 10 μg/mL, and could inhibit only 50% of the Tf-TfR binding. Thus, it was suggested that the TfR436 antibody should be excellent in terms of inhibition of the Tf-TfR binding.

Example 3: Comparison between intracellular iron content and growth-inhibiting effect of TfR436 antibody in AML clinical specimen

In order to verify the relationship between an intracellular iron content and the growth-inhibiting effect obtained by exposure to a TfR436 antibody in an AML clinical specimen, the following test was carried out.

(1) Growth-inhibiting effect of TfR436 antibody on AML clinical specimen

The concentration causing 50% cell growth inhibition (GI50 value) of a TfR436 antibody obtained by exposing an AML clinical specimen to the TfR436 antibody was calculated. Specifically, an AML clinical specimen (bone marrow or peripheral blood) was subjected to mononuclear cell isolation, and the obtained cells were suspended in MethoCult H4534 Classic without EPO (STEMCELL #ST-04534) to a cell density of 5000 cells/90 μL, and were then seeded on a 96-well plate. A TfR436 antibody was diluted with an RPMI-1640 Medium (Thermo Fisher #11875119), and 10 μL each of the diluted antibody was added to the AML clinical specimen-derived cells to final concentrations of 5000, 1000, 500, 100, 50, 10, 5 and 1 ng/mL, and 0 (not added). The thus obtained mixtures were cultured at 37°C in a 5% $CO_2$ incubator for 7 days, and thereafter, 100 μL of CellTiter-Glo Luminescent Cell Viability Assay (Promega #G7572) was added to and blended with the culture in each well. Ten minutes later, fluorescence was detected using a luminometer. The well, to which only the medium had been added, was used as a blank. The mean value in the TfR436 antibody-not-added well was set at 100%, and the growth percentage in each antibody concentration was calculated. After that, using XLfit, the GI50 value was calculated.

(2) Measurement of intracellular iron content in AML clinical specimen

Using an AML clinical specimen, the intracellular iron content was measured. Specifically, $1.0 \times 10^7$ cells were collected from the AML clinical specimen, and were then washed with DPBS three times. 250 μL of Lysis M Reagent (Roche #04719 956001) and 2.5 μL of 6 N-HCl were added to and blended with the cell pellets, and the obtained mixture was then left at rest at room temperature for 1 hour. Thereafter, the reaction mixture was centrifuged, and the recovered supernatant was used in iron quantification. By performing the same operations as those described above, a K562 cell line was prepared as a standard sample, and a frozen stock was then produced. Iron quantification was carried out according to metalloassay iron measurement LS (ferrozine method) (Metallogenics #FE31M). Besides, every time the quantification of the intracellular iron in the AML clinical specimen, quantification of the intracellular iron in the K562 cell line was also carried out, and the comparison value was calculated.

(3) Relationship between intracellular iron content and growth-inhibiting effect of TfR436 antibody

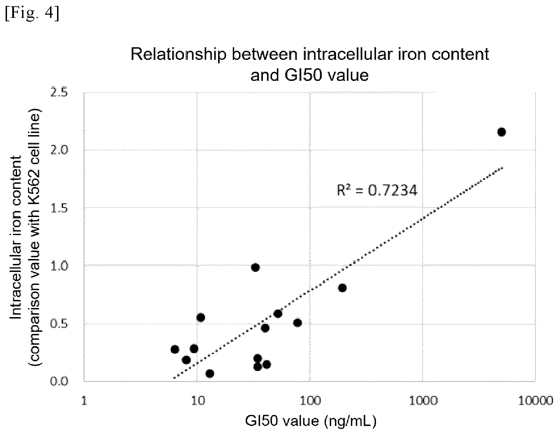

The relationship between the intracellular iron content in each AML clinical specimen and the concentration causing 50% cell growth inhibition (GI50 value) of a TfR436 antibody is shown in Fig. 4.

As a result, the intracellular iron content in the AML clinical specimen was correlated with the GI50 value, and when the intracellular iron content was a low value, the GI50 value also tended to become a low value. From these results, it was suggested that when the intracellular iron content in the AML clinical specimen is a low value, the TfR436 antibody would be likely to exhibit sufficient pharmacological effects.

Example 4: Comparison between intracellular iron content and growth-inhibiting effect of TfR436 antibody in cell lines

In order to demonstrate the relationship between an intracellular iron content and the growth-inhibiting effect obtained by exposure to a TfR436 antibody, which was verified in Example 3, the following test was carried out using cell lines.

(1) Measurement of intracellular iron content in cell lines

Using Kasumi-1 (American Type Culture Collection, ATCC #CRL-2724), HL60 (JCRB Cell Bank, #JCRB0085), KO52 (JCRB Cell Bank, #JCRB0123), and CCRF-CEM (ATCC #CCL-119), the intracellular iron content in each cell line was measured. Specifically, Kasumi-1 and HL60 were each suspended in an RPMI1640 + 20% FBS medium, KO52 was suspended in an Alpha-MEM + 10% FBS medium, and CCRF-CEM was suspended in an RPMI1640 + 10% FBS medium, so that the concentration of each cell line became $1.0 \times 10^5$ cells/mL, and thereafter, 20 mL of the obtained suspension was seeded in a T75 flask. A negative control monoclonal antibody was added thereto to a final concentration of 5 μg/mL, and the thus obtained mixture was then cultured at 37°C in a 5% $CO_2$ incubator. Ninety-six hours later, the cells were recovered, and were then washed with PBS. Thereafter, 250 μL of Lysis M Reagent and 2.5 μL of 6 N-HCl were added to and blended with $1.0 \times 10^7$ cells of each cell line, and the obtained mixture was then left at rest at room temperature for 1 hour. Thereafter, the reaction mixture was centrifuged, and the recovered supernatant was used in iron quantification. As a standard sample, the K562 (ATCC #CCL-243) cell line was cultured in an RPMI1640 + 10% FBS medium and was then recovered, as with other cell lines, and a supernatant of the thus prepared K562 (ATCC #CCL-243) cell line was used in iron quantification. Such iron quantification was carried out according to the metalloassay iron measurement LS (ferrozine method). The measured iron content was divided by the number of cells used as a sample, so as to obtain an intracellular iron content per cell. Thereafter, the comparison value of the intracellular iron content in each cell line to the intracellular iron content in the K562 cell line was calculated.

(2) Growth-inhibiting effect of TfR436 antibody on cell lines

Using Kasumi-1, HL60, KO52 and CCRF-CEM, the concentration causing 50% cell growth inhibition (GI50 value) of a TfR436 antibody obtained by exposing each cell line to the TfR436 antibody was calculated. Specifically, individual cell lines were each suspended in various types of optimal cell culture media to result in a cell concentration of 10000 cells/50 μL, and thereafter, the obtained suspensions were each seeded on a 96-well plate. The TfR436 antibody and a negative control monoclonal antibody were each diluted with the same media as those described above to 9 levels, so that a common ratio became 3.16 from a final concentration of 5000 ng/mL. These antibody-added media and antibody-not-added media were added in an amount of 50 μL each to individual cell lines. The thus obtained mixtures were cultured at 37°C in a 5% $CO_2$ incubator for 96 hours, and thereafter, 20 μL of CellTiter 96 AQueous One Solution Cell Proliferation Assay (Promega #G3580) was added to and blended with the culture in each well. The obtained mixtures were left at rest at 37°C in a 5% $CO_2$ incubator for 4 hours, and the absorbance at 505 nm was then detected. The well, to which only the medium had been added, was used as a blank. The mean value in the antibody-not-added well was set to be a growth percentage of 100%, and the growth percentage in each antibody concentration was calculated. After that, using MasterPlex ReaderFit, the GI50 value was calculated.

(3) Intracellular iron content and growth-inhibiting effect of TfR436 antibody in each cell line

The intracellular iron content and the concentration causing 50% cell growth inhibition (GI50 value) of a TfR436 antibody in each cell line are shown in Table 1.

As a result, according to the relationship between the intracellular iron content and the GI50 value of a TfR436 antibody of Example 3, as shown in Fig. 4, all of the verified cell lines exhibited a low intracellular iron content and a low GI50 value. It was suggested that the TfR436 antibody would be likely to exhibit sufficient pharmacological effects on these cell lines each having a low intracellular iron content.

[Table 1]

| Cell Line | Comparison Value with K562 | GI50 (ng/mL) |
|-----------|---------------------------|--------------|
| Kasumi-1 | 0.43 | 39.0 |
| HL60 | 0.21 | 43.5 |
| KO52 | 0.33 | 84.0 |
| CCRF-CEM | 0.13 | 37.5 |

Example 5: Antitumor effects of TfR436 antibody on Kasumi-1 subcutaneous transplantation models

In order to verify the pharmacological effects of a TfR436 antibody on cell line-derived xenograft (CDX) models, the following test was carried out using subcutaneous transplantation models comprising the Kasumi-1 verified in Example 4.

The acute myelogenous leukemia cell line Kasumi-1 was cultured in an RPMI-1640 supplemented with 10% FBS and 1% P/S. The cells obtained by expanded culture were centrifuged at 1,000 rpm for 5 minutes, and thereafter, pellets were recovered and were then suspended in an antibiotic- and serum-free RPMI-1640 medium to prepare a cell suspension with a cell concentration of $2 \times 10^8$/mL. Under cooling on ice, an equal amount of Matrigel was added to the resulting cells, and was mixed therewith by inverting. The thus obtained mixture (100 μL) was transplanted into the subcutis on the right ventral side of each mouse. The number of cells transplanted was $1 \times 10^7$/mouse.

The mean tumor volume of the transplanted mice was measured over time, and on Day 21 after the transplantation, in which the tumor volume tended to increase, stratified randomized allocation was carried out based on the tumor volume, using the statistical analysis system for biological experiment data EXSUS (Version 8.1, EP Croit Co., Ltd.). Regarding group structure, there are 4 groups, and n = 8 in each group.

The applied dose was set to be 10 mL/kg, and a negative antibody group (10 mg/kg) was used as a control group. Regarding administration groups, the TfR436 antibody was administered at a dose of 1, 3, and 10 mg/kg, once a week, two times in total. After completion of the administration, the tumor was measured using a Vernier caliper twice a week, and the tumor volume of each group was obtained. The antitumor effects of the TfR436 antibody were determined based on the tumor volume.

The tumor volume was calculated according to the following equation:

$$\text{Tumor volume} = (\text{minor axis})^2 \text{ x major axis x } 0.5.$$

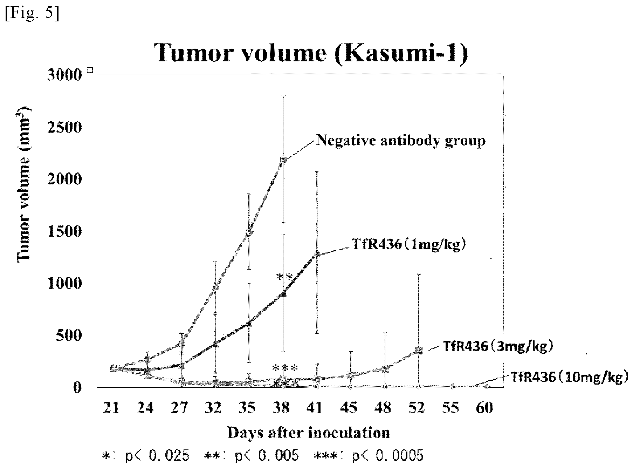

Changes over time in the mean values of tumor volume are shown in Fig. 5. Dose-dependent antitumor effects were confirmed at the concentration of the TfR436 antibody that was 1 mg/kg or more, and all of the administration groups were confirmed to have a significant difference from the negative antibody group (Group 2: p = 0.0012; Groups 3 and 4: P < 0.0001). In particular, in the 10 mg/kg TfR436 antibody administration group, tumors disappeared from all of the mice, and even on the test termination date (Day 60 after the transplantation), the regrowth of the tumor was not confirmed.

Example 6: Antitumor effects of TfR436 antibody on HL60 subcutaneous transplantation models

In order to verify the pharmacological effects of a TfR436 antibody on cell line-derived xenograft (CDX) models, the following test was carried out using subcutaneous transplantation models comprising the HL60 verified in Example 4.

The acute promyelocytic leukemia cell line HL60 was cultured in an RPMI-1640 supplemented with 10% FBS and 1% P/S. The cells obtained by expanded culture were centrifuged at 1,000 rpm for 5 minutes, and thereafter, pellets were recovered and were then suspended in an antibiotic- and serum-free RPMI-1640 medium to prepare a cell suspension with a cell concentration of $2 \times 10^8$/mL. Under cooling on ice, an equal amount of Matrigel was added to the resulting cells, and was mixed therewith by inverting. The thus obtained mixture (100 μL) was transplanted into the subcutis on the right ventral side of each mouse. The number of cells transplanted was $1 \times 10^7$/mouse.

The mean tumor volume of the transplanted mice was measured over time, and on Day 9 after the transplantation, in which the tumor volume tended to increase, stratified randomized allocation was carried out based on the tumor volume, using the statistical analysis system for biological experiment data EXSUS (Version 8.1, EP Croit Co., Ltd.). Regarding group structure, there are 5 groups, and n = 5 in each group.

The applied dose was set to be 10 mL/kg. Citrate was administered to a negative control group, and a TfR436 antibody was administered at a dose of 0.3 1, 3, and 10 mg/kg to administration groups, once a week, four times in total. After completion of the administration, the tumor was measured using a Vernier caliper twice a week, and the tumor volume of each group was obtained. The antitumor effects of the TfR436 antibody were determined based on the tumor volume.

The tumor volume was calculated according to the following equation:

$$\text{Tumor volume} = (\text{minor axis})^2 \text{ x major axis x } 0.5.$$

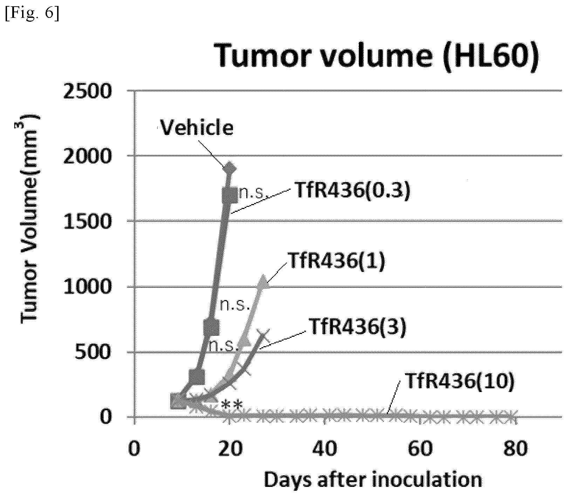

Changes over time in the mean values of tumor volume are shown in Fig. 6. Significant dose-dependent antitumor effects were confirmed at the concentration of the TfR436 antibody that was 1 mg/kg or more. In particular, in the 10 mg/kg TfR436 antibody administration group, tumors disappeared from all of the mice, and even 49 days after completion of the administration (Day 79 after the transplantation), the regrowth of the tumor was not confirmed. A statistically significant difference was confirmed in the 10 mg/kg TfR436 antibody administration group (p = 0.0011).

Example 7: Life-extending effect of TfR436 antibody on KO52 caudal vein transplantation models

In order to verify the pharmacological effects of a TfR436 antibody on cell line-derived xenograft (CDX) models, the following test was carried out using caudal vein transplantation models comprising the KO52 verified in Example 4.

The acute myeloblastic leukemia cell line KO52 was cultured in an Alpha-MEM supplemented with 10% FBS and 1% P/S. The cells obtained by expanded culture were centrifuged at 1,000 rpm for 10 minutes, and thereafter, pellets were recovered and were then suspended in a normal saline for injection to prepare a cell suspension with a cell concentration of $2.5 \times 10^7$/mL. The prepared cell suspension was stored in ice and was brought in a breeding room. Each mouse was placed in a 29G Myjector, and 200 $\mu$L of the cell suspension was then transplanted from the caudal vein of the mouse. The number of cells transplanted was $5 \times 10^6$/mouse.

Three days after the transplantation of the cells, stratified randomized allocation was carried out based on the body weight, using the statistical analysis system for biological experiment data EXSUS (Version 8.1, EP Croit Co., Ltd.). Mice, in which defects such as the leakage of the cell solution had been found upon the cell transplantation, were excluded from grouping in advance. Regarding group structure, there are 3 groups, and n = 6 in each group. After completion of the grouping, the mice were observed at a frequency of 2 or 3 times a week. When signs of dying, such as weakness or decreased body temperature, were found as a result of observation of the general physical conditions, it was determined to be a humanitarian endpoint, and the concerned mouse was subjected to euthanasia.

The applied dose was set to be 10 mL/kg. Citrate was administered to a negative control group, and a TfR436 antibody was administered at a dose of 10 mg/kg to administration groups, once two weeks, two times in total. After completion of the second administration, to one of the two administration groups, the TfR436 antibody was administered again on the 4th week, once two weeks, so that administration was carried out a total of 4 times to the one administration group.

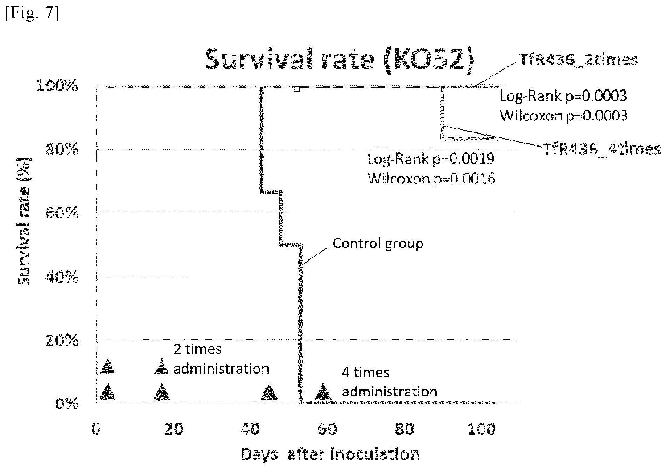

During the test period, the mice were observed, and life-and-death judgment was carried out. On Day 104 after the transplantation, the test was terminated, and statistical analysis was carried out based on the survival time of the mice. As a result, by comparing the TfR436 antibody administration groups with the negative control group, in the case of the two times administration group, p = 0.0003 in Log-Rank test, and p = 0.0003 in Wilcoxon test. In the 4 times administration group, p = 0.0019 in Log-Rank test, and p = 0.0016 in Wilcoxon test. Thus, significant life-extending effects was confirmed in each administration group (Fig. 7). Besides, as a result of autopsy, it was determined that one death case found in the 4 times administration group was not influenced by transplantation of KO52, but that the death was caused by spontaneously generated thymoma that was specific to scid mice.

Example 8: Life-extending effect of TfR436 antibody on CCRF-CEM intravenous transplantation models

In order to verify the pharmacological effects of a TfR436 antibody on cell line-derived xenograft (CDX) models, the following test was carried out using intravenous transplantation models comprising the CCRF-CEM verified in Example 4.

The T cell acute leukemia cell line CCRF-CEM was cultured in an RPMI-1640 supplemented with 10% FBS and 1% P/S. The cells obtained by expanded culture were centrifuged at 1,000 rpm for 5 minutes, and thereafter, pellets were recovered and were then suspended in a normal saline for injection to prepare a cell suspension with a cell concentration of $2.5 \times 10^7$/mL. The prepared cell suspension (200 $\mu$L) was transplanted from the caudal vein of the mouse. The number of cells transplanted was $5 \times 10^6$/mouse.

Three days after the transplantation of the cells, stratified randomized allocation was carried out based on the body weight, using the statistical analysis system for biological experiment data EXSUS (Version 8.1, EP Croit Co., Ltd.). Mice, in which defects such as the leakage of the cell solution had been found upon the cell transplantation, were excluded from grouping in advance. Regarding group structure, there are 5 groups, and n = 10 in each group.

Regarding administration, initial administration was carried out on Day 3 after transplantation (the day of grouping) and Day 7 after the transplantation, and according to the schedule shown in Table 2, administration was carried out on each group, 5 times in total. Until termination of the test, the body weight of each mouse was measured at a frequency of twice a week. The general physical conditions of the mice were observed, twice a week until 30 days after the transplantation, and then, from Day 31, every day until 97 days after the transplantation, which was termination of the test. The body weight of each mouse was measured at a frequency of twice a week, and the percentage of the measured body weight to the body weight at the time of grouping was calculated. When the body weight reduction percentage exceeded 20%, it was determined to be a humanitarian endpoint, and the concerned mouse was subjected to euthanasia. Even other than the body weight reduction, when signs such as weakness or decreased body temperature were found as a result of the observation of the general physical conditions, it was determined to be a humanitarian endpoint.

[Table 2]

| Group | Treatment | Dose (mg/kg) | n | Dosing Frequency | Total Number of Doses | Administration Route | Initial Administration Date |
|---|---|---|---|---|---|---|---|
| 1 | Vehicle (PBS) | 0 | 10 | Twice/week | 5 times | Intravenous administration | Day 3 |
| 2 | TfR436 | 1 | 10 | Twice/week | 5 times | Intravenous administration | Day 3 |
| 3 | TfR436 | 10 | 10 | Twice/week | 5 times | Intravenous administration | Day 3 |
| 4 | TfR436 | 1 | 10 | Twice/week | 5 times | Intravenous administration | Day 7 |
| 5 | TfR436 | 10 | 10 | Twice/week | 5 times | Intravenous administration | Day 7 |

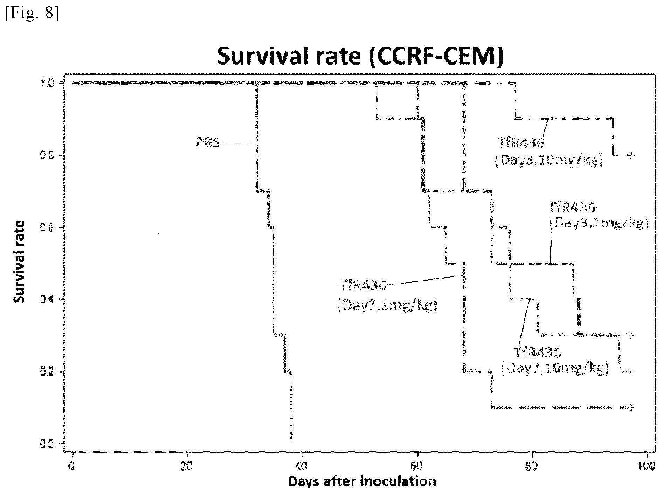

In comparison with the negative control PBS group, a significant life-extending effect (Wilcoxon test) was confirmed in all of the administration groups. Regarding the high-dose administration group (Day 3, 10 mg/kg) shown in Fig. 8 and Table 3, in which initial administration was carried out on Day 3 after the transplantation, 80% of the mice survived at the time of termination of the test, and thus, a significant life-extending effect was confirmed. In addition, even in low-dose administration groups, TfR436 significantly prolonged the survival period of the mice, and exhibited an excellent life-extending effect.

[Table 3]

| Group | Assay | Adjusted p value | Mark |
|---|---|---|---|
| 1 PBS | - | - | - |
| 2 (Day 3; 1 mg/kg) | Log-Rank | 0.0005 | *** |
| | Wilcoxon | p < 0.0001 | *** |
| 3 (Day 3; 10 mg/kg) | Log-Rank | p < 0.0001 | *** |
| | Wilcoxon | p < 0.0001 | *** |
| 4 (Day 7; 1 mg/kg) | Log-Rank | 0.1069 | n. s. |
| | Wilcoxon | 0.0074 | ** |
| 5 (Day 7, 10 mg/kg) | Log-Rank | 0.0052 | ** |
| | Wilcoxon | 0.0001 | *** |
| n. s.: No significant difference<br>*: p < 0.05<br>**: p < 0.01<br>***: p < 0.001 | | | |

## Claims

1. A method for determining the medicinal effect or sensitivity of an anti-human transferrin receptor antibody having an action to inhibit the binding between human transferrin and a human transferrin receptor, wherein the method uses an intracellular iron content as an indicator.

2. The method according to claim 1, which is a method for determining the medicinal effect or sensitivity by utilizing the correlation of the intracellular iron content with the medicinal effect of the antibody.

3. The method according to claim 1 or 2, wherein the medicinal effect or sensitivity of the antibody is determined based on the relative value to the iron content in a reference sample.

4. The method according to any one of claims 1 to 3, wherein when the intracellular iron content is lower than the reference value, the medicinal effect or sensitivity of the antibody is determined to be high.

5. The method according to any one of claims 1 to 4, wherein the medicinal effect or sensitivity of the antibody to cancer is determined.

6. The method according to claim 5, wherein the cancer is blood cancer.

7. The method according to claim 5 or 6, wherein the cancer is acute myelogenous leukemia (AML).

8. The method according to any one of claims 1 to 7, wherein the antibody is an antibody recognizing the amino acids at positions 629 to 633 of a human transferrin receptor.

9. The method according to any one of claims 1 to 8, wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

10. The method according to any one of claims 1 to 9, wherein the antibody is an antibody having a heavy chain as set forth in SEQ ID NO: 7 and a light chain as set forth in SEQ ID NO: 8.

[Fig. 1]

[Fig. 2]

[Fig. 3]

[Fig. 4]

Relationship between intracellular iron content and GI50 value

$R^2 = 0.7234$

[Fig. 5]

Tumor volume (Kasumi-1)

*: p< 0.025　**: p< 0.005　***: p< 0.0005

[Fig. 6]

Tumor volume (HL60)

[Fig. 7]

Survival rate (KO52)

[Fig. 8]

Survival rate (CCRF-CEM)

## INTERNATIONAL SEARCH REPORT

International application No.

**PCT/JP2021/037296**

### A. CLASSIFICATION OF SUBJECT MATTER

*G01N 33/84*(2006.01)i; *C07K 14/705*(2006.01)i; *C07K 16/28*(2006.01)i; *C07K 16/46*(2006.01)i; *G01N 33/15*(2006.01)i
FI: G01N33/84 Z; C07K14/705; C07K16/46; C07K16/28 ZNA; G01N33/15 Z

According to International Patent Classification (IPC) or to both national classification and IPC

### B. FIELDS SEARCHED

Minimum documentation searched (classification system followed by classification symbols)
G01N33/84; C07K14/705; C07K16/28; C07K16/46; G01N33/15

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); MEDLINE/EMBASE (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2008-303168 A (JAPAN HEALTH SCIENCES FOUNDATION) 18 December 2008 (2008-12-18) claims, paragraphs [0033], [0035] | 1-4, 8-10 |
| Y | WO 2020/105621 A1 (PERSEUS PROTEOMICS INC.) 28 May 2020 (2020-05-28) claims | 1-4, 8-10 |
| A | JP 2014-93958 A (UNIVERSITY OF MIYAZAKI) 22 May 2014 (2014-05-22) entire text, all drawings | 1-10 |
| A | US 2013/0171061 A1 (YANG, Ming-Hua) 04 July 2013 (2013-07-04) entire text, all drawings | 1-10 |
| A | JP 2008-508904 A (RAVEN BIOTECHNOLOGIES, INC.) 27 March 2008 (2008-03-27) entire text, all drawings | 1-10 |
| A | JP 2006-517032 A (GEORGETOWN UNIVERSITY) 13 July 2006 (2006-07-13) entire text, all drawings | 1-10 |

☑ Further documents are listed in the continuation of Box C.    ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2021** | **30 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 227 686 A1

**INTERNATIONAL SEARCH REPORT**

International application No.

**PCT/JP2021/037296**

**C.    DOCUMENTS CONSIDERED TO BE RELEVANT**

| Category* | Citation of document, with indication, where appropriate, of the relevant passages | Relevant to claim No. |
|---|---|---|
| A | WO 2015/108172 A1 (METALLOGENICS CO., LTD.) 23 July 2015 (2015-07-23)<br>entire text, all drawings | 1-10 |
| A | US 2019/0101549 A1 (INSTITUTE NATIONAL DE LA SANTE ET DE LA RESEARCHE MEDICALE) 04 April 2019 (2019-04-04)<br>entire text, all drawings | 1-10 |
| A | RUDOLPH, Natalie S. et al. Regulation of K562 Cell Transferrin Receptors by Exogenous Iron. Journal of Cellular Physiology, March 1985, vol. 122, no. 3, pp. 441-450<br>entire text, all drawings | 1-10 |
| A | NEIVEYANS, Madeline et al. A recycling anti-transferrin receptor-1 monoclonal antibody as an efficient therapy for erythroleukemia through target up-regulation and antibody-dependent cytotoxic effector functions, mAbs, 18 February 2019., vol. 11, no. 3, pp. 593-605<br>entire text, all drawings | 1-10 |
| A | SHIMOSAKI, Shunsuke et al. Development of a complete human IgG monoclonal antibody to transferrin receptor 1 targeted for adult T-cell leukemia/lymphoma. Biochemical and Biophysical Research Communications, 08 February 2017, vol. 485, pp. 144-151<br>entire text, all drawings | 1-10 |
| A | CREPIN, Ronan et al. Development of Human Single-Chain Antibodies to the Transferrin Receptor that Effectively Antagonize the Growth of Leukemias and Lymphomas. Cancer Research, 08 June 2010, vol. 70, no. 13, pp. 5497-5506<br>entire text, all drawings | 1-10 |

Form PCT/ISA/210 (second sheet) (January 2015)

**INTERNATIONAL SEARCH REPORT**

International application No.

**PCT/JP2021/037296**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/JP2021/037296**

| Patent document cited in search report | | | Publication date (day/month/year) | Patent family member(s) | | | Publication date (day/month/year) |
|---|---|---|---|---|---|---|---|
| JP | 2008-303168 | A | 18 December 2008 | (Family: none) | | | |
| WO | 2020/105621 | A1 | 28 May 2020 | (Family: none) | | | |
| JP | 2014-93958 | A | 22 May 2014 | (Family: none) | | | |
| US | 2013/0171061 | A1 | 04 July 2013 | TW | 201325623 | A | |
| JP | 2008-508904 | A | 27 March 2008 | US | 2006/0039908 | A1 | |
| | | | | entire text | | | |
| | | | | WO | 2005/121179 | A2 | |
| | | | | EP | 1765868 | A2 | |
| | | | | CA | 2569692 | A1 | |
| | | | | CN | 101432305 | A | |
| | | | | AU | 2005252699 | A1 | |
| | | | | KR | 10-2007-0042959 | A | |
| JP | 2006-517032 | A | 13 July 2006 | US | 2004/0241088 | A1 | |
| | | | | entire text | | | |
| | | | | WO | 2004/066946 | A2 | |
| | | | | EP | 1595142 | A2 | |
| | | | | CA | 2513769 | A1 | |
| WO | 2015/108172 | A1 | 23 July 2015 | US | 2016/0356796 | A1 | |
| | | | | entire text | | | |
| | | | | EP | 3096143 | A1 | |
| | | | | CN | 105917234 | A | |
| US | 2019/0101549 | A1 | 04 April 2019 | WO | 2017/153475 | A1 | |
| | | | | entire text | | | |
| | | | | EP | 3427059 | A1 | |

Form PCT/ISA/210 (patent family annex) (January 2015)

## REFERENCES CITED IN THE DESCRIPTION

**Patent documents cited in the description**

- WO 2014073641 A
- WO 2020105621 A
- WO 9202190 A
- US 5530101 A
- US 5585089 A
- US 5693761 A
- US 5693792 A
- US 5714350 A
- US 5777085 A
- WO 9201047 A
- WO 9220791 A
- WO 9306213 A
- WO 9311236 A
- WO 9319172 A
- WO 9501438 A
- WO 9515388 A
- WO 9710354 A
- US 20080193453 A

**Non-patent literature cited in the description**

- **P. PONKA ; C. N. LOK.** The transferrin receptor: role in health and disease. *Int. J. Biochem. Cell Biol,* 1999, vol. 31, 1111-1137
- **D. R. RICHARDSON ; P. PONKA.** The molecular mechanisms of the metabolism and transport of iron in normal and neoplastic cells. *Biochim. Biophys. Acta,* 1997, vol. 1331, 1-40
- **DANIELS TR. DELGADO T ; RODRIGUEZ JA ; HELGUERA G ; PENICHET ML.** The transferrin receptor part I: Biology and targeting with cytotoxic antibodies for the treatment of cancer. *Clinical Immunology,* 2006, vol. 121, 144-158
- **J. E. LEVY ; O. JIN ; Y. FUJIWARA ; F. KUO ; N. C. ANDREWS.** Transferrin receptor is necessary for development of erythrocytes and the nervous system. *Nat. Genet.,* 1999, vol. 21, 396-399
- **KHATUN R ; WU Y ; KANENISHI K ; UENO M ; TANAKA S ; HATA T ; SAKAMOTO H.** Immunohistochemical study of transferrin receptor expression in the placenta of pre-eclamptic pregnancy. *Placenta,* 2003, vol. 24 (8-9), 870-6
- **WINTER et al.** *Immunol. Today,* 1993, vol. 14, 43-46
- **HASHIMOTO-GOTOH, T ; MIZUNO, T ; OGASAHARA, Y ; DNA KAGAWA, M.** An oligodeoxyribonucleotide-directed dual amber method for site-directed mutagenesis. *Gene,* 1995, vol. 152, 271-275
- **ZOLLER, MJ ; SMITH, M.** Oligonucleotide-directed mutagenesis of DNA fragments cloned into M13 vectors. *Methods Enzymol.,* 1983, vol. 100, 468-500
- **KRAMER, W ; DRUTSA, V ; JANSEN, HW ; KRAMER, B ; PFLUGFELDER, M ; FRITZ, HJ.** The gapped duplex DNA approach to oligonucleotide-directed mutation construction. *Nucleic Acids Res.,* 1984, vol. 12, 9441-9456
- **KRAMER W ; FRITZ HJ.** Oligonucleotide-directed construction of mutations via gapped duplex DNA Methods. *Enzymol.,* 1987, vol. 154, 350-367
- **KUNKEL, TA.** Rapid and efficient site-specific mutagenesis without phenotypic selection. *Proc Natl Acad Sci USA,* 1985, vol. 82, 488-492
- **KABAT, E. A. et al.** Sequences of Proteins of Immunological Interest. U. S. Department of Health and Human Services, 1991
- **A. WRIGHT ; S. L. MORRISON.** *J. Immunol.,* 1998, vol. 160, 3393-3402
- **K. MOTMANS et al.** *Eur. J. Cancer Prev.,* 1996, vol. 5, 512-5199
- **R. P. JUNGHANS et al.** *Cancer Res.,* 1990, vol. 50, 1495-1502
- **R. W. MALONE et al.** *Proc. Natl. Acad. Sci. USA,* 1989, vol. 86, 6007
- **P. L. FELGNER et al.** *Proc. Natl. Acad. Sci. USA,* 1987, vol. 84, 7413
- **F. L. GRAHAM ; A. J. VAN DER EB.** *Virology,* 1973, vol. 52, 456-467